# FASCICULE DE BREVET EUROPEEN

(11) **EP 1 281 391 B1**
(45) Date de publication et mention de la délivrance du brevet: **21.06.2006**
(21) Numéro de dépôt: 02291756.1
(22) Date de dépôt: 11.07.2002
(51) Int. Cl.: A61Q 17/04, A61K 8/30

(54) **Filtres UV organiques insolubles de type aryl vinylène cétone et leur utilisation en cosmétique**
Organische unlösliche UV-Filter des Typs Arylvinylenketon und deren Verwendung in der Kosmetik
Organic insoluble UV filters of the aryl vinylene ketone type and their use in cosmetics

(30) Priorité: 18.07.2001 FR 0109634
(43) Date de publication de la demande: 05.02.2003
(73) Titulaire: L'ORÉAL, 75008 Paris (FR)
(72) Inventeur: Richard, Hervé, 93420 Villepinte (FR)
(74) Mandataire: Casalonga, Axel

(56) Documents cités:
- EP-A- 1 093 797
- WO-A-00/15329
- WO-A-00/35415
- DE-A- 10 027 950
- DE-A- 19 952 410

## Description

La présente invention concerne des composés organiques insolubles filtrant le rayonnement UV de type aryl vinylène cétone, des compositions cosmétiques les contenant ainsi que leur utilisation à titre d'agents filtrant le rayonnement UV pour protéger des matériaux sensibles au rayonnement UV, la peau, les cheveux ou pour contrôler la couleur de la peau.

On sait que les radiations lumineuses de longueurs d'onde comprises entre 280 nm et 400 nm permettent le brunissement de l'épiderme humain et que les rayons de longueurs d'onde comprises entre 280 et 320 nm, connus sous la dénomination d'UV-B, provoquent des érythèmes et des brûlures cutanées qui peuvent nuire au développement du bronzage naturel. Pour ces raisons, ainsi que pour des raisons esthétiques, il existe une demande constante de moyens de contrôle de ce bronzage naturel en vue de contrôler ainsi la couleur de la peau. Il convient donc de filtrer ce rayonnement UV-B.

On sait également que les rayons UV-A, de longueurs d'onde comprises entre 320 nm et 400 nm, qui provoquent le brunissement de la peau, sont susceptibles d'induire une altération de celle-ci, notamment dans le cas d'une peau sensible ou d'une peau continuellement exposée au rayonnement solaire. Les rayons UV-A provoquent en particulier une perte d'élasticité de la peau et l'apparition de rides conduisant à un vieillissement prématuré. Ils favorisent le déclenchement de la réaction érythémateuse ou amplifient cette réaction chez certains sujets et peuvent même être à l'origine de réactions phototoxiques ou photo-allergiques. Ainsi, pour des raisons esthétiques et cosmétiques telles que la conservation de l'élasticité naturelle de la peau par exemple, de plus en plus de personnes désirent contrôler l'effet des rayons UVA sur leur peau. Il est donc souhaitable de filtrer aussi le rayonnement UV-A.

Un grand nombre de composés ont déjà été proposés comme filtres solaires, essentiellement sous la forme de filtres organiques solubles ou de composés minéraux insolubles. Ces filtres doivent pouvoir absorber ou bloquer les rayons nocifs du soleil tout en restant inoffensifs pour l'utilisateur.

A cet égard, et afin de limiter les éventuels risques d'allergie cutanée associés à l'utilisation de filtres organiques solubles, on utilise depuis un certain temps de plus en plus de composés minéraux insolubles tels que l'oxyde de zinc ou encore l'oxyde de titane. Cependant, ces composés minéraux peuvent présenter l'inconvénient d'être sensibles au rayonnement solaire. Par ailleurs, à quantités équivalentes, ces composés minéraux sont moins efficaces dans la protection UV que les filtres organiques.

Un certain nombre de composés organiques à groupe aryl vinylène cétone ont déjà été proposés comme filtres solaires. On peut citer à titre d'exemples les benzylidène camphres décrits dans le brevet FR 2 528 420, les benzylidène-nor-camphres décrits dans le brevet EP 0 693 471, les benzylidène cétotricyclodécanes décrits dans le brevet EP 0 694 521, les benzylidène cinéoles décrits au 16^{ème} IFSCC Congress de New York (1990), les benzylidène quinuclidinones décrites dans les brevets EP 0 576 974 et EP 0 714 880, les styryl cétones décrites dans le brevet JP 04 134 042, les para xylidène cétones décrites dans le brevet JP 04 134 041, les alpha benzylidène cétones décrites dans le brevet JP 04 134 043, les alpha acétyl cinnamates décrits dans le brevet JP 11 080 091, les benzylidène cycloalcanones décrites dans le brevet FR 2 395 023, les benzylidène hydantoïnes décrites dans le brevet JP 01 158 090, les benzylidène furanones décrites dans les brevets EP 0 390 683 et FR 2 638 354, les chalcones décrites dans les brevets FR 2 555 167 et FR 2 608 150, ainsi que les alpha pyrones décrites dans le brevet JP 04 290 882.

Aucun de ces documents ne mentionne cependant l'utilisation de ces filtres sous forme de particules insolubles de très petite taille, c'est-à-dire une taille de l'ordre du micron ou inférieure au micron.

On connaît également, par les documents WO 95/22959, WO 97/03643, WO 99/66896 et WO 00/78277, des filtres ou mélanges de filtres UV organiques insolubles ou difficilement solubles pouvant comprendre, entre autres, des dérivés du type oxalanilide, triazine, triazole, amide vinylique, cinnamamide, benzimidazole sulfonique, acide cinnamique, diphénylacrylate, camphre, etc., micronisés sous forme de particules de taille moyenne comprise entre 0,01 et 2 µm. Certains de ces filtres, en particulier les dérivés de benzimidazole sulfonique, présentent des variations de solubilité à température élevée qui conduisent à une recristallisation importante dans le support. Un tel phénomène peut se traduire par une perte d'efficacité des filtres et une détérioration des propriétés cosmétiques des compositions les contenant.

La demanderesse, suite à d'importantes recherches dans le domaine de la photoprotection, a découvert de nouveaux composés organiques insolubles contenant un ou plusieurs groupements aryl vinylène cétone capables d'absorber à la fois dans l'UV-A et dans l'UV-B, se trouvant sous forme de fines particules de taille moyenne comprise entre 10 nm et 5 µm, qui permettent de protéger efficacement la peau, les lèvres ou les cheveux ainsi que d'autres matériaux photosensibles contre les effets défavorables du rayonnement UV.

Au sens où on l'utilise dans la présente invention, le terme aryl vinylène cétone englobe tous les dérivés aryl vinylène contenant un carbonyle en alpha de la double liaison et ne contenant pas de groupement amide.

On entend par composé insoluble, au sens de la présente invention, un composé ayant une solubilité dans l'eau inférieure à 0,1 % en poids et une solubilité inférieure à 1 % en poids dans la plupart des solvants organiques comme l'huile de paraffine, les benzoates d'alcools gras et les triglycérides d'acides gras, par exemple le Miglyol® 812 commercialisé par la société DYNAMIT NOBEL. Cette solubilité, définie à 70 °C comme la quantité de produit en solution dans le solvant à l'équilibre avec un excès de solide en suspension, peut facilement être évaluée au laboratoire.

Ces composés organiques insolubles permettent de surmonter les inconvénients des filtres organiques solubles ou insolubles et des filtres minéraux insolubles de l'art antérieur. Ces matériaux sont des filtres solaires plus efficaces que les filtres minéraux de l'état de la technique, puisqu'ils cumulent deux mécanismes de filtrage, à savoir l'absorption du rayonnement UV par les chromophores organiques et la dispersion-réflexion de la lumière par les surfaces des particules. Ils sont donc tout à fait appropriés pour la préparation de compositions cosmétiques destinées à la protection de la peau et des cheveux contre le rayonnement solaire. De plus, outre leurs propriétés filtrantes et dispersantes, les composés organiques insolubles selon l'invention présentent une bonne stabilité chimique et photochimique.

La présente invention a par conséquent pour objet des composés organiques insolubles filtrant le rayonnement UV du type aryl vinylène cétone correspondant à l'une des formules (I) et (II) ci-après et qui se présentent sous forme de particules ayant une taille moyenne comprise entre 10 nm et 5 µm.

L'invention a également pour objet des compositions cosmétiques contenant au moins un composé organique insoluble de formule (I) ou (II), sous forme de particules ayant une taille moyenne comprise entre 10 nm et 5 µm.

L'invention a également pour objet l'utilisation de ces composés insolubles comme agents filtrant le rayonnement UV-A et UV-B, par exemple pour la protection de matériaux sensibles au rayonnement UV, en particulier au rayonnement solaire, pour contrôler la couleur de la peau ou pour la préparation de médicaments destinés à prévenir les effets néfastes du rayonnement UV-A et UV-B.

Les composés organiques insolubles du type aryl vinylène cétone filtrant le rayonnement UV ayant une taille moyenne comprise entre 10 nm et 5 µm sont choisis parmi ceux correspondant à l'une des formules (I) et (II) suivantes : 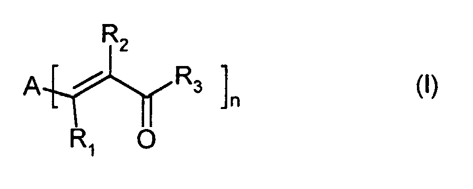 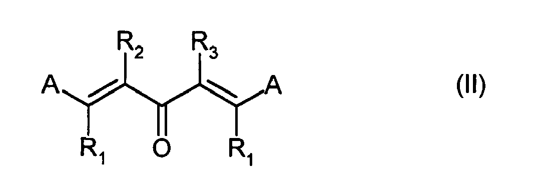 dans lesquelles :
n = 1 ou 2,
A, dans la formule (I) lorsque n=1 ou dans la formule (II), est un radical aryle choisi parmi les formules (a) à (d) suivantes, ou dans la formule (I) lorsque n=2, est un radical choisi parmi les formules (e) à (h) suivantes : 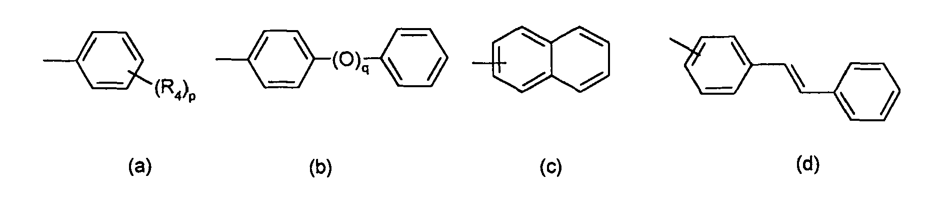 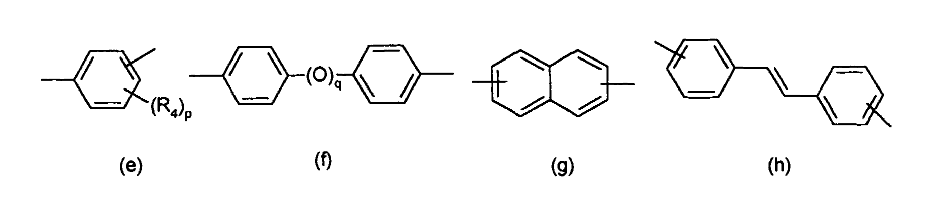
dans lesquelles :
chacun des symboles R₄ représente indépendamment un groupe OH, un atome d'halogène, un groupe alkyle en C₁₋₆, linéaire ou ramifié et contenant éventuellement un atome de silicium, un groupe alcoxy en C₁₋₆, linéaire ou ramifié et contenant éventuellement un atome de silicium, un groupe alcoxycarbonyle en C₁₋₅, linéaire ou ramifié, ou un groupe alkylsulfonamide en C₁₋₆, linéaire ou ramifié et contenant éventuellement un atome de silicium ou une fonction aminoacide,
p représente un nombre entier compris entre 0 et 4 inclus,
q représente 0 ou 1,
R₁ représente l'hydrogène ou un groupe OH,
R₂ représente l'hydrogène, un groupe alkyle en C₁₋₆, linéaire ou ramifié et contenant éventuellement un atome de silicium, un groupe cyano, un groupe alkylsulfonyle en C₁₋₆, un groupe phénylsulfonyle,
R₃ représente un groupe alkyle en C₁₋₆, linéaire ou ramifié et contenant éventuellement un atome de silicium ou un groupe phényle pouvant former un bicycle et éventuellement substitué par un ou deux radicaux R₄,
ou R₂ et R₃ forment ensemble un reste hydrocarboné en C₂₋₁₀ monocyclique, bicyclique ou tricylique, éventuellement interrompu par un ou des atomes d'azote, de soufre et d'oxygène et pouvant contenir un autre carbonyle, et éventuellement substitué par un groupe alkylsulfonamide en C₁-C₈, linéaire ou ramifié, et contenant éventuellement un atome de silicium ou une fonction aminoacide ; à condition que lorsque n=1, R₂ et R₃ ne forment pas un noyau camphre.

A titre d'exemples de composés de formule (I) dans laquelle n=1, insolubles, filtrant le rayonnement UV, ayant une taille moyenne de particules comprise entre 10 nm et 5 µm, on peut mentionner les familles suivantes :
- Styryl Cétone (Kao JP 04 134 042) telle que la 1-(3,4-diméthoxy phényl)-4,4-diméthyl-pent-1-èn-3-one : 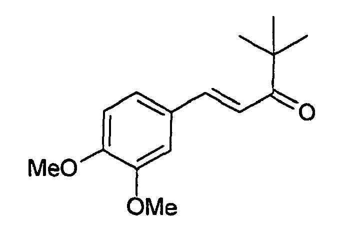
- Benzylidène Cinéole (E. Mariani et al, 16^{th} IFSCC Congress, New York (1990)) tel la 1,3,3-triméthyl-5-(4-méthoxy-benzylidène)-2-oxa-bicyclo[2.2.2]octan-6-one : 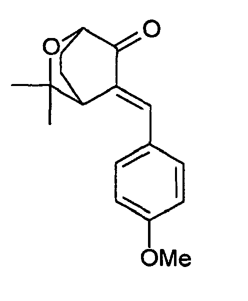
- Benzylidène Chromanone (Kao JP 04 134 043) telle que la 3-(4-méthoxy-benzylidène)-2,3,4a,8a-tétrahydro-chromèn-4-one : 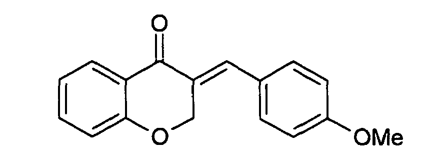
- Benzylidène Thiochromanone (Kao JP 04 134 043) telle que la 3-(4-méthoxy-benzylidène)-2,3,4a,8a-tétrahydro-chromèn-4-thione 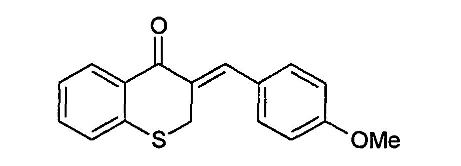
- Benzylidène Quinuclidinone (Merck EP 0 576 974) telle que la 4-méthoxy benzylidène-1-aza-bicyclo[2.2.2]octan-3-one: 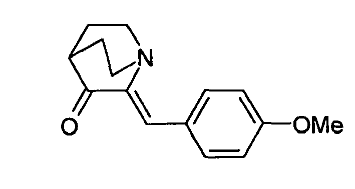
- Benzylidène Cycloalcanone (Henkel FR 2 395 023) telle que les 2-(4-méthoxy-benzylidène)-cyclopentanone et 2-(4-méthoxy-benzylidène)-cyclohexanone : 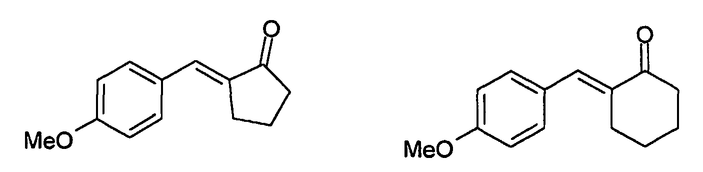
- Benzylidène Hydantoïne (Ajinomoto JP 01 158 090) telle que la 5-(3,4-diméthoxy-benzylidène)-imidazolidine-2,4-dione : 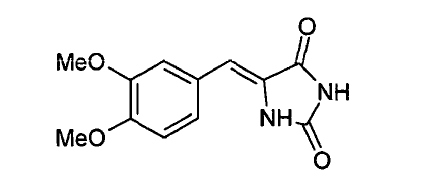
- Benzylidène Indanone (Kao JP 04 134 043) telle que la 2-(4-méthoxy-benzylidène)-indan-1-one : 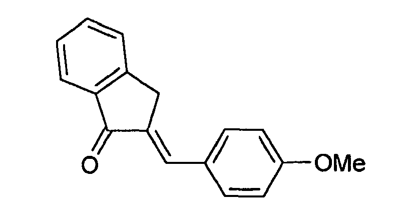
- Benzylidène Tétralone (Kao JP 04 134 043) telle que la 2-(4-méthoxy-benzylidène)-3,4-dihydro-2H-naphthalen-1-one : 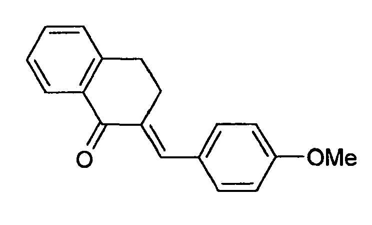
- Benzylidène Furanone (L'Oréal EP 0 390 683) telle que la 4-(4-méthoxy-benzylidène)-2,2,5,5-tétraméthyl-dihydro-furan-3-one : 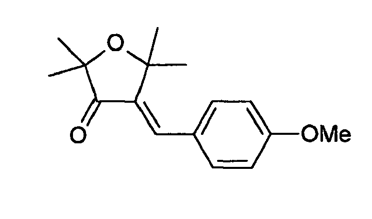
- Benzylidène Benzofuranone (Kao JP 04 134 041) telle que la 2-benzylidène-benzofuran-3-one : 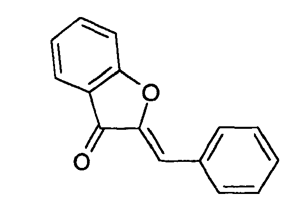
- Benzylidène Indanedione telle que la 2-(3,5-di-tert-butyl-4-hydroxybenzylidène)-indan-1,3-dione : 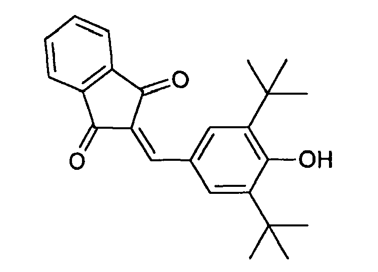
- Benzylidène Benzothiofuranone (Kao JP 04,134,043) telle que la 2-benzylidène-benzo[b]thiophen-3-one : 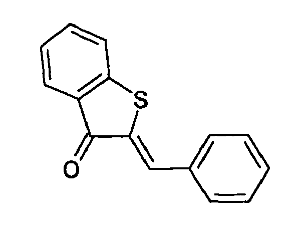
- Benzylidène Barbiturique tel que la 5-(4-méthoxy-benzylidène)-1,3-diméthyl-pyrimidine-2,4,6-trione : 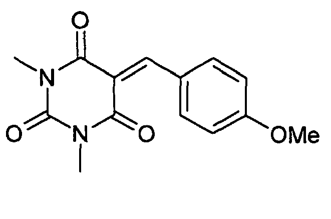
- Benzylidène Pyrazolone telle que la 4-(4-méthoxy-benzylidène)-5-méthyl-2-phényl-2,4-dihydro-pyrazol-3-one : 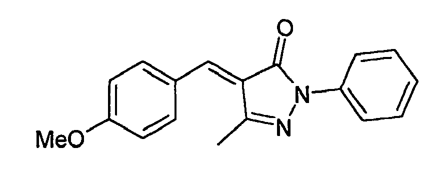
- Benzylidène Imidazolone telle que la 5-(4-méthoxy-benzylidène)-2-phényl-3,5-dihydro-imidazol-4-one 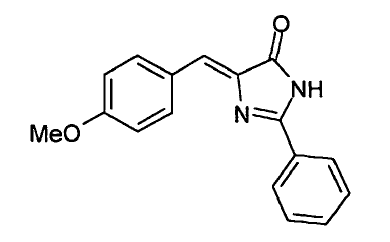
- Chalcone telle que la 1-(2-hydroxy-4-méthoxy-phényl)-3-phénylpropènone : 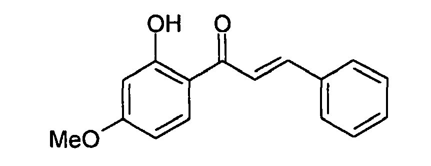
- Benzylidène One (forme tautomère filtrante des dibenzoylméthanes ; L'Oréal FR 2 506 156) telle que la 3-hydroxy-1-(2-hydroxy-4-méthoxy-phényl)-3-phényl-propènone :

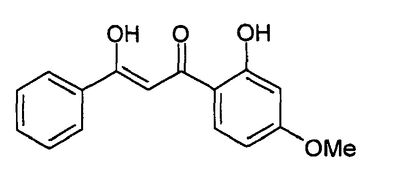

A titre d'exemples de composés de formule (I) dans laquelle n=2 insolubles, filtrant le rayonnement UV, ayant une taille moyenne de particules comprise entre 10 nm et 5 µm, on peut mentionner les familles suivantes :
- Phénylène bis méthylidène-nor-camphre (Merck EP 0 693 471) tel que la 1,4-phénylène-bis-{3-méthylidène-bicyclo[2.2.1]heptan-2-one} : 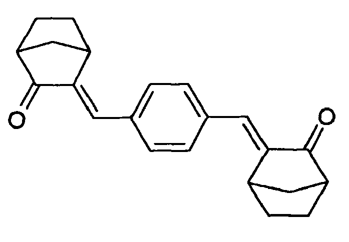
- Phénylène bis méthylidène camphre (L'Oréal FR 2 528 420) telle que la 1,4-phénylène-bis-{3-méthylidène-1,7,7-triméthyl-bicyclo [2.2.1]heptan-2-one} : 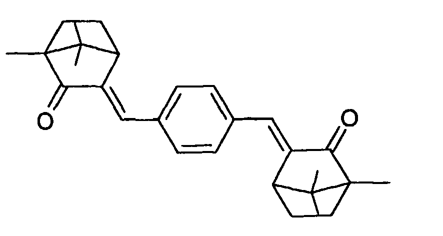
ou la 1,3-phénylène-bis-{3-méthylidène-1,7,7-triméthyl-bicyclo [2.2.1]heptan-2-one}: 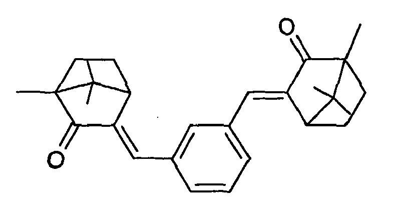
- Phénylène bis méthylidène camphre sulfonamide (L'Oréal FR 2 529 887) tel le 1,4-phénylène-bis-3,3'-méthylidène camphre-10,10'-sulfonamide d'éthyle ou de 2-éthylhexyle : 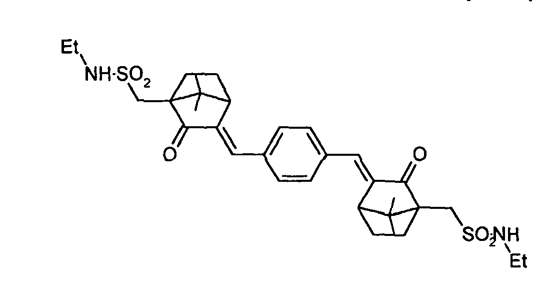
ou 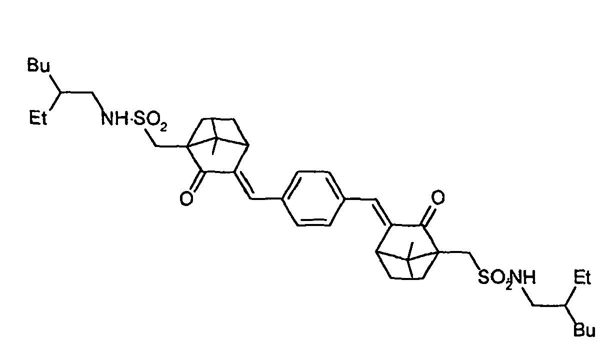
- Phénylène bis méthylidène cinéole (E. Mariani et al, 16^{th} IFSCC Congress, New York (1990)) tel la 1,4-phénylène-bis-{5-méthylidène-3,3-diméthyl-2-oxa-bicyclo[2.2.2]octan-6-one} : 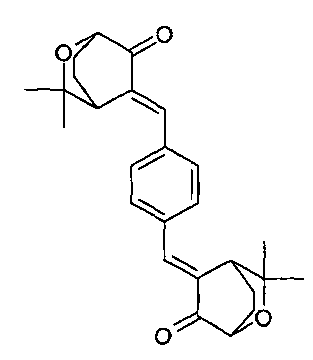
- Phénylène bis méthylidène cétotricyclodécane (Merck EP 0 694 521) tel la 1,4-phénylène-bis-(octahydro-4,7-méthano-6-indèn-5-one) : 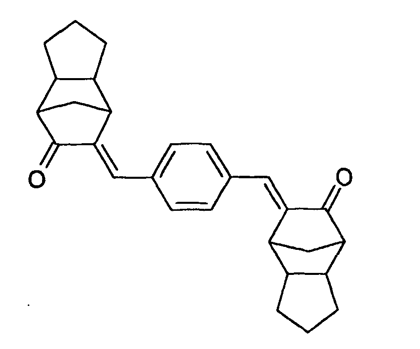
- Phénylène bis alkylène cétone (Kao JP 04 134 041) telle que la 1,4-phénylène-bis-(4,4-diméthyl-pent-1-èn-3-one) : 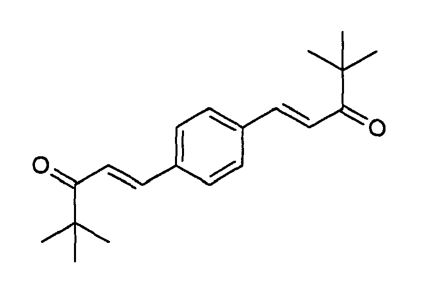
- Phénylène bis méthylidène furanone (L'Oréal FR 2 638 354) telle que la 1,4-phénylène-bis-(4-méthylidène-2,2,5,5-tétraméthyldihydrofuran-3-one) : 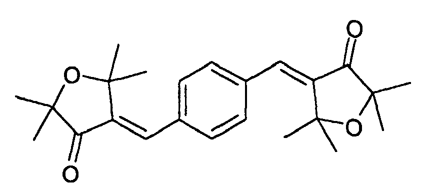
- Phénylène bis méthylidène quinuclidinone (Merck EP 0 714 880) telle que la 1,4-phénylène-bis-{2-méthylidène-1-aza-bicyclo [2.2.2]octan-3-one}:

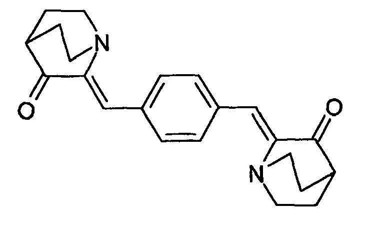

A titre de composés de formule (II), on peut mentionner les familles suivantes :
- bis benzylidène cycloalcanone telle que la 2,5-dibenzylidènecyclopentanone : 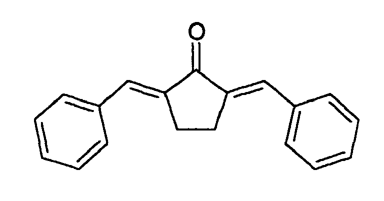
- gamma pyrone (Kao JP 04 290 882) telle que la 2,6-bis-(3,4-diméthoxy-phényl)-pyran-4-one :

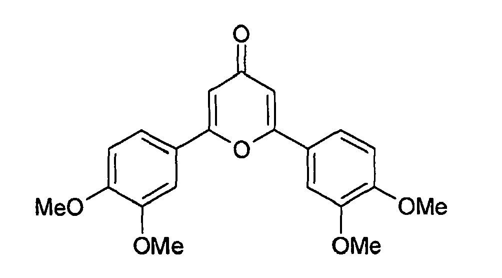

Parmi ces composés organiques insolubles filtrant le rayonnement UV, on préfère tout particulièrement les composés de formule (I) dans laquelle n=2.

Comme indiqué ci-dessus, la taille moyenne des particules insolubles des composés organiques décrits ci-dessus est comprise entre 10 nm et 5 µm.

Les particules ont de préférence une taille moyenne comprise entre 10 nm et 2 µm, et plus préférentiellement entre 20 nm et 2 µm.

D'une manière générale, la taille moyenne des particules correspondra au diamètre moyen de la distribution en nombre.

La taille moyenne des particules peut être déterminée par toute méthode classique telle que les méthodes optiques (diffusion quasi-élastique
ou diffusion laser), les méthodes par centrifugation ou les méthodes de visualisation microscopique et analyse d'image.

Les filtres organiques insolubles selon l'invention peuvent être amenés sous la forme particulaire ayant la taille moyenne souhaitée par tout moyen approprié, tel que broyage à sec ou en milieu solvant, tamisage, atomisation, micronisation, pulvérisation ou tout procédé chimique tel que précipitation par émulsion ou dilution.

Les filtres organiques insolubles selon l'invention peuvent être en particulier obtenus par un procédé de broyage de particules grossières en présence d'un ou de plusieurs agents tensio-actifs appropriés permettant d'améliorer la dispersion des particules ainsi obtenues dans les formulations cosmétiques.

Un exemple de procédé de réduction de taille des particules de filtres organiques insolubles est décrit dans les documents WO 95/22959 et WO 97/03643. L'appareil de broyage utilisé selon ces documents peut être un broyeur à jet, à billes, à vibration ou à marteau et de préférence un broyeur à haute vitesse d'agitation ou un broyeur à impact et plus particulièrement un broyeur à billes rotatives, un broyeur vibrant, un broyeur à tube ou un broyeur à tige.

Les composés organiques insolubles filtrant les UV peuvent être utilisés dans des compositions cosmétiques, en particulier des compositions protectrices de l'épiderme humain ou des cheveux, des compositions anti-solaires ou des produits de maquillage.

Ces compositions cosmétiques contiennent généralement de 0,1 à 15 % en poids, de préférence de 0,2 à 10 % en poids, par rapport au poids total de la composition cosmétique, de composés organiques insolubles filtrant le rayonnement UV.

Les compositions cosmétiques, et en particulier anti-solaires, de la présente invention peuvent contenir en outre un ou plusieurs filtres solaires organiques additionnels, actifs dans le domaine des UV-A et/ou UV-B, différents des filtres insolubles décrits ci-dessus.

Ces filtres solaires peuvent être notamment choisis parmi les dérivés cinnamiques, les dérivés de dibenzoylméthane ; les dérivés salicyliques ; les dérivés du benzylidènecamphre ; les dérivés de triazine tels que ceux décrits dans les brevets ou demandes de brevet US 4 367 390, EP 0 863 145, EP 0 517 104, EP 0 570 838, EP 0 796 851, EP 0 775 698, EP 0 878 469, EP 0 933 376, EP 0 507 691, EP 0 507 692, EP 0 790 243, EP 0 944 624 et US 4 724 137 ; les dérivés de la benzophénone ; les dérivés de β,β'-diphénylacrylate ; les dérivés de phénylbenzimidazole ; les dérivés anthraniliques ; les dérivés d'imidazolines ; les dérivés de méthylène-bis-(hydroxyphénylbenzotriazole) tels que ceux décrits dans les brevets ou demandes de brevet US 5 237 071, US 5 166 355, GB 2 303 549, DE 19726184 et EP 0 893 119 ; les dérivés de l'acide p-aminobenzoïque ; les polymères hydrocarbonés filtres et les silicones filtres tels que ceux décrits notamment dans la demande WO 93/04665 ; les dimères dérivés d'α-alkyl styrène tels que décrits dans la demande DE 198 55 649 ; les 4,4-diaryl butadiènes tels que décrits dans les demandes EP 0 967 200 et DE 197 55 649 ; ainsi que les mélanges de ces filtres.

On peut citer à titre d'exemples de tels filtres solaires additionnels actifs dans l'UV-A et/ou l'UV-B, les composés suivants désignés par leur nom INCI, ainsi que leur mélanges :

### dérivés d'acide para-aminobenzoïque :

- PABA
- Ethyl PABA
- Ethyl Dihydroxypropyl PABA
- Ethylhexyl Dimethyl PABA vendu notamment sous le nom commercial ESCALOL 507 par ISP,
- Glyceryl PABA,
- PEG-25 PABA vendu sous le nom commercial UVINUL P25 par BASF,

### dérivés salicyliques :

- Homosalate vendu sous le nom commercial EUSOLEX HMS par RONA/EM INDUSTRIES,
- Ethylhexyl Salicylate vendu sous le nom commercial NEO HELIOPAN OS par HAARMANN ET REIMER,
- Dipropyleneglycol Salicylate vendu sous le nom commercial DIPSAL par SCHER,
- TEA Salicylate vendu sous le nom commercial NEO HELIOPAN TS par HAARMANN ET REIMER,

### dérivés de dibenzoylméthane :

- Butyl Methoxydibenzoylmethane vendu notamment sous le nom commercial PARSOL 1789 par HOFFMANN LAROCHE,
- Isopropyl Dibenzoylmethane,

### dérivés cinnamiques :

- Ethylhexyl Methoxycinnamate vendu notamment sous le nom commercial PARSOL MCX par HOFFMANN LAROCHE,
- Isopropyl Methoxycinnamate
- Isoamyl Methoxycinnamate vendu sous le nom commercial NEO HELIOPAN E 1000 par HAARMANN ET REIMER,
- Cinoxate,
- DEA Methoxycinnamate,
- Diisopropyl Methylcinnamate,
- Glyceryl Ethylhexanoate Dimethoxycinnamate

### dérivés de β,β-diphénylacrylate :

- Octocrylene vendu notamment sous le nom commercial UVINUL-539 par BASF

### dérivés de benzophénone :

- Benzophenone-1 vendue sous le nom commercial UVINUL 400 par BASF,
- Benzophenone-2 vendue sous le nom commercial UVINUL D-50 par BASF,
- Benzophenone-3 ou Oxybenzone vendue sous le nom commercial UVINUL M-40 par BASF,
- Benzophenone-4 vendue sous le nom commercial UVINUL MS-40 par BASF,
- Benzophenone-5,
- Benzophenone-6 vendue sous le nom commercial HELISORB 11 par NORQUAY,
- Benzophenone-8 vendue sous le nom commercial SPECTRA-SORB UV-24 par AMERICAN CYANAMID
- Benzophenone-9 vendue sous le nom commercial UVINUL DS-49 par BASF,
- Benzophénone-12

### dérivés du benzylidène camphre :

- Benzylidene Camphor Sulfonic Acid fabriqué sous le nom MEXORYL SL par CHIMEX,
- Camphor Benzalkonium Methosulfate fabriqué sous le nom MEXORYL SO par CHIMEX,
- Terephthalylidene Dicamphor Sulfonic Acid fabriqué sous le nom MEXORYL SX par CHIMEX,
- Polyacrylamidomethyl Benzylidene Camphor fabriqué sous le nom MEXORYL SW par CHIMEX,

### dérivés de phénylbenzimidazole :

- Phenylbenzimidazole Sulfonic Acid vendu notamment sous le nom commercial EUSOLEX 232 par MERCK
- Benzimidazilate vendu sous le nom commercial NEO HELIOPAN AP par HAARMANN ET REIMER,

### dérivés de triazine :

- Anisotriazine vendue sous le nom commercial TINOSORB S par CIBA GEIGY
- Ethylhexyl triazone vendue notamment sous le nom commercial UVINUL T150 par BASF,
- Diéthylhexyl Butamido Triazone vendue sous le nom commercial UVASORB HEB par SIGMA 3V,
- 2,4,6-tris (4'-aminobenzalmalonate de di-isobutyle) s-triazine,

### dérivés de phénylbenzotriazole :

- Drometrizole Trisiloxane vendu sous le nom commercial SILATRIZOLE par RHODIA CHIMIE,
- Methylene bis-Benzotriazolyl Tetramethylbutylphenol vendu sous forme solide sous le nom commercial MIXXIM BB/100 par FAIRMOUNT CHEMICAL ou sous forme micronisée en dispersion aqueuse sous le nom commercial TINOSORB M par CIBA SPECIALTY CHEMICALS,

### dérivés anthraniliques :

- Menthyl Anthranilate vendu sous le nom commercial NEO HELIOPAN MA par HAARMAN ET REIMER,

### dérivés d'imidazolines :

- Ethylhexyl Dimethoxybenzylidene Dioxoimidazoline Propionate

### dérivés du benzalmalonate :

- Polyorganosiloxane à fonctions benzalmalonate vendu sous le nom commercial PARSOL SLX par HOFFMANN LAROCHE

Les filtres UV organiques plus particulièrement préférés sont choisis parmi les composés suivants :
- Ethylhexyl Salicylate,
- Butyl Methoxydibenzoylmethane,
- Ethylhexyl Methoxycinnamate,
- Octocrylene,
- Phenylbenzimidazole Sulfonic Acid,
- Terephthalylidene Dicamphor Sulfonic Acid,
- Benzophenone-3,
- Benzophenone-4,
- Benzophenone-5,
- Benzimidazilate,
- Anisotriazine,
- Ethylhexyl Triazone,
- Diethylhexyl Butamido Triazone,
- 2,4,5-tris (4'-aminobenzalmalonate de di-isobutyle) s-triazine,
- Methylene bis-Benzotriazolyl Tetramethylbutylphenol,
- Drometrizole Trisiloxane,
et leurs mélanges.

Les compositions cosmétiques selon l'invention peuvent contenir en outre des agents de bronzage et/ou de brunissage artificiel de la peau (agents autobronzants) tels que la dihydroxyacétone (DHA).

Les compositions cosmétiques selon l'invention peuvent contenir en outre un ou plusieurs pigments minéraux et en particulier des nanopigments d'oxydes métalliques, enrobés ou non, comme par exemple les nanopigments d'oxyde de titane sous forme amorphe ou cristallisée (rutile et/ou anatase), d'oxyde de fer, d'oxyde de zinc, d'oxyde de zirconium ou d'oxyde de cérium. Ces nanopigments ont une taille moyenne de particules comprise entre 5 nm et 100 nm, de préférence entre 10 nm et 50 nm et sont tous des agents photoprotecteurs UV connus.

Ces nanopigments peuvent être enrobés par des agents d'enrobage connus comme par exemple l'alumine et/ou le stéarate d'aluminium.

De tels nanopigments enrobés ou non enrobés sont décrits par exemple dans les demandes de brevets EP-A-0 518 772 et EP-A-0 518 773.

Les compositions cosmétiques peuvent contenir en outre des adjuvants de formulation usuels tels que les corps gras, les solvants organiques physiologiquement acceptables, les silicones, les agents tensioactifs, les polymères anioniques, cationiques, non ioniques, amphotères ou leurs mélanges, les agents épaississants, les agents antioxydants, les agents opacifiants, les agents stabilisants, les agents anti-mousse, les parfums, les agents conservateurs, les charges, les agents séquestrants, les agents propulseurs, les agents d'ajustement du pH, les colorants et leurs mélanges.

Elles peuvent contenir en outre un ou plusieurs principes actifs cosmétiques choisis par exemple parmi les agents adoucissants, les hydroxyacides, les vitamines, les agents hydratants, les agents émollients, les agents anti-radicalaires, les antagonistes de substance P, les agents anti-inflammatoires et des mélanges de ces composés.

Les corps gras peuvent être constitués par une huile ou une cire ou leurs mélanges, et ils comprennent également les acides gras, les alcools gras et les esters d'acides gras. Les huiles peuvent être choisies parmi les huiles animales, végétales, minérales ou de synthèse et notamment parmi l'huile de vaseline, l'huile de paraffine, les huiles de silicone, volatiles ou non, les isoparaffines, les polyoléfines, les huiles fluorées et perfluorées. De même, les cires peuvent être choisies parmi les cires animales, fossiles, végétales, minérales ou de synthèse connues en soi.

Parmi les solvants organiques, on peut citer les alcools et polyols inférieurs tels que l'éthanol, l'isopropanol, le propylèneglycol, la glycérine et le sorbitol.

Les épaississants peuvent être choisis notamment parmi les gommes de guar et celluloses, modifiées ou non, telles que la gomme de guar hydroxypropylée, la méthylhydroxyéthylcellulose, l'hydroxypropylméthylcellulose ou encore l'hydroxyéthylcellulose.

Bien entendu l'homme du métier veillera à choisir ce ou ces éventuels composés complémentaires et/ou leurs quantités de manière à ce que les propriétés avantageuses intrinsèques à l'invention, et en particulier l'insolubilité des agents filtrant le rayonnement UV, ne soient pas altérées par la ou les adjonctions envisagées.

Les compositions de l'invention peuvent être préparées selon les techniques bien connues de l'homme du métier, en particulier celles destinées à la préparation d'émulsions de type huile-dans-eau ou eau-dans-huile.

Cette composition peut se présenter en particulier sous forme d'émulsion, simple ou complexe (H/E, E/H, H/E/H ou E/H/E) telle qu'une crème, un lait, un gel ou un gel-crème, de poudre ou de bâtonnet solide, et peuvent éventuellement être conditionnée en aérosol et se présenter sous forme de mousse ou de spray.

Lorsqu'il s'agit d'une émulsion, la phase aqueuse de celle-ci peut comprendre une dispersion vésiculaire non ionique préparée selon des procédés connus (Bangham, Standish and Watkins, J. Mol. Biol. 13, 238 (1965), FR 2 315 991 et FR 2 416 008).

La composition cosmétique de l'invention peut être utilisée comme composition protectrice de l'épiderme humain ou des cheveux contre les rayons ultraviolets, comme composition anti-solaire ou comme produit de maquillage.

Lorsque la composition cosmétique selon l'invention est utilisée pour la protection de l'épiderme humain contre les rayons UV, ou comme composition antisolaire, elle peut se présenter sous forme de suspension ou de dispersion dans des solvants ou des corps gras, sous forme de dispersion vésiculaire non ionique ou encore sous forme d'émulsion, de préférence de type huile-dans-eau, telle qu'une crème ou un lait, sous forme de pommade, de gel, de gel crème, de bâtonnet solide, de stick, de poudre, de mousse aérosol ou de spray.

Lorsque la composition cosmétique selon l'invention est utilisée pour la protection des cheveux contre les rayons UV, elle peut se présenter sous forme de shampooing, de lotion, de gel, d'émulsion, de dispersion vésiculaire non ionique et constituer par exemple une composition à rincer, à appliquer avant ou après shampoooing, avant ou après coloration ou décoloration, avant, pendant ou après permanente ou défrisage, une lotion ou un gel coiffants ou traitants, une lotion ou un gel pour le brushing ou la mise en plis, une composition de permanente ou de défrisage, de coloration ou de décoloration des cheveux.

Lorsque la composition est utilisée comme produit de maquillage des cils, des sourcils ou de la peau, tel que crème de traitement de l'épiderme, fond de tein, bâton de rouge à lèvres, fard à paupières, fard à joues, mascara ou ligneur encore appelé "eye-liner", elle peut se présenter sous forme solide ou pâteuse, anhydre ou aqueuse, comme des émulsions huile-dans-eau ou eau-dans-huile, des dispersions vésiculaires non ioniques ou encore des suspensions.

A titre indicatif, pour les formulations anti-solaires conformes à l'invention, qui présentent un support de type émulsion huile-dans-eau, la phase aqueuse représente généralement de 50 à 95 % en poids, de préférence de 70 à 90 % en poids, par rapport à l'ensemble de la formulation, la phase huileuse de 5 à 50 % en poids, de préférence de 10 à 30 % en poids, par rapport à l'ensemble de la formulation, et le ou les (co)émulsionnant(s) de 0,5 à 20 % en poids, de préférence de 2 à 10 % en poids, par rapport à l'ensemble de la formulation.

Les agents filtrants insolubles de la présente invention peuvent également être utilisés pour protéger des matériaux, comme des verres organiques ou minéraux ou les matières plastiques, sensibles au rayonnement UV, en particulier au rayonnement solaire. Les particules sont alors appliquées sur ou incorporées dans le matériau à protéger en une quantité efficace.

L'invention a aussi pour objet un procédé non-thérapeutique de protection de la peau et/ou des cheveux contre le rayonnement ultraviolet, en particulier le rayonnement solaire, qui consiste à appliquer sur la peau ou les cheveux une quantité efficace de la composition cosmétique définie ci-dessus, ou d'un composé de formule (I) ou (II) tel que défini ci-avant.

L'invention a enfin pour objet un procédé non-thérapeutique de contrôle de la variation de couleur de la peau due au rayonnement UV, qui consiste à appliquer sur la peau une quantité efficace de la composition cosmétique définie ci-dessus, ou d'un composé de formule (I) ou (II) tel que défini ci-avant.

Les exemples suivants illustrent l'invention.

### Exemples de composés

### Exemple 1

### Synthèse du 1,4-phénylène-bis-3,3'-méthylidène camphre-10,10' sulfonamide d'éthyle

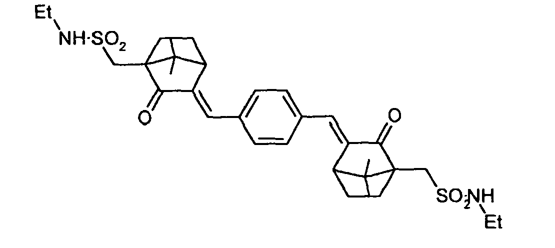

Le sel disodique de l'acide 1,4-phénylène-bis-3,3'-méthylidène camphre-10,10'-sulfonique (12,1g, 0,02 mole) est mis en suspension dans 50 ml de DMF sec. La température monte jusque 32°C. On ajoute ensuite de l'éthylamine (4,5g, 0,1 mole) puis de la triéthylamine (4,9g, 0,044 mole). On laisse sous agitation pendant 1 heure. On verse le mélange réactionnel refroidi dans 100 ml d'eau. Le solide obtenu est filtré, lavé à l'eau puis dans un mélange acétone/eau pour donner le 1,4-phénylène-bis-3,3'-méthylidène camphre-10,10'-sulfonamide d'éthyle (5g, Rendement : 80%) sous forme d'une poudre blanche (Pf 165°C) :
- UV (solution dans DMSO/CH₂Cl₂) : λₘₐₓ = 342 nm, εₘₐₓ = 40350, E1% = 654
- Analyse élémentaire pour C₃₂H₄₄N₂O₆S₂
   - Calculé : C62,31 H7,19 N4,54 O15,56 S10,40
   - Trouvé : C62,78 H7,14 N4,21 O16.12 S10,62

### Exemple 2

### Synthèse du 1,4-phénylène-bis-3,3'-méthylidène camphre-10,10'-sulfonamide de 2-éthylhexyle

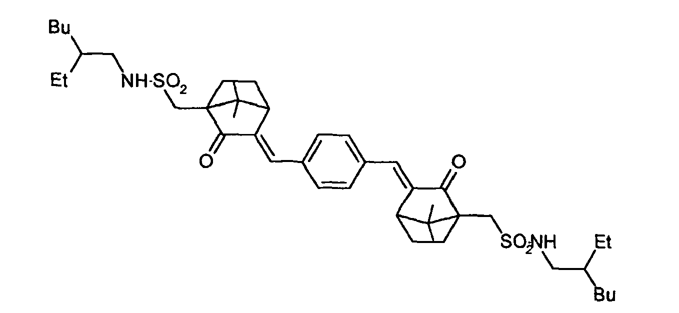

Le sel disodique de l'acide 1,4-phénylène-bis-3,3'-méthylidène camphre-10,10'-sulfonique (12,1g, 0,02 mole est mis en suspension dans 50ml de DMF sec. La température monte jusque 32°C. On ajoute ensuite le 2-éthylhexylamine (5,6g, 0,044 mole) puis de la triéthylamine (4,9g, 0,044 mole). On laisse sous agitation pendant 1 heure. On extrait au dichlorométhane, on lave à l'eau la phase organique, on la sèche et on évapore le solvant. L'huile très épaisse obtenue est cristallisée dans l'éthanol pour donner le 1,4-phénylène-bis-3,3'-méthylidène camphre-10,10'-sulfonamide de 2-éthyl hexyle (30g, Rendement : 64%) sous forme d'une poudre blanche (Pf 165°C) :
- UV (solution dans CH₂Cl₂) : λₘₐₓ = 342 nm, εₘₐₓ = 41 000 , E1% = 522
- Analyse élémentaire pour C₄₄H₆₈N₂O₆S₂
   - Calculé : C67,35 H8,67 N3,51 012,25 S8,16
   - Trouvé : C67,40 H8,55 N3,21 012,67 S7,70

### Exemple 3

### Synthèse du 1,3-phénylène-bis-(3-méthylidène camphre)

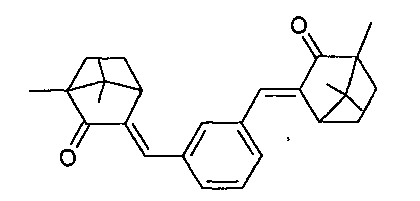

Dans un ballon équipé d'un Dean Stark, on place le camphre (33,5g, 0,22mole) et le tert-butylate de potassium en poudre (24,7g, 0,22 mole) dans 250ml de toluène sec. Le mélange est porté au reflux et laissé à cette température pendant 1 heure. On refroidit vers 40°C et on ajoute ensuite goutte à goutte en 1 heure l'iso phtaldéhyde (13,4g, 0,1 mole) dissous dans 200ml de toluène sec. On porte le mélange 3 heures 30 minutes au reflux avec élimination de l'eau formée. Le mélange est refroidi et on le verse dans la glace contenant 25ml d'acide chlorhydrique concentré. La phase organique est séparée, lavée à l'eau, séchée sur sulfate de sodium, filtrée et le solvant évaporé. La masse obtenue est purifiée sur colonne de silice (éluant :CH₂CL₂), puis recristallisée dans le cyclohexane pour donner le 1,3-phénylène-bis-(3-méthylidène camphre) (10,4g, Rendement 25%) sous forme d'une poudre blanche (Pf 152°C).
- UV (solution dans CHCl₃) : λₘₐₓ = 295 nm, εₘₐₓ = 41 500 , E1% = 1 040
- Analyse élémentaire pour C₂₈H₃₄O₂
   - Calculé : C83,54 H8,51 07,95
   - Trouvé : C83,48 H8,50 08,25

### Exemple 4

### Synthèse du 1,4-phénylène-bis-(méthylidène cétotricyclodécane)

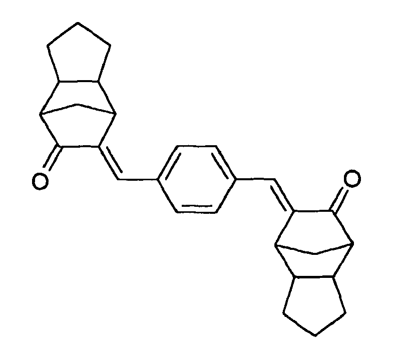

Dans un ballon équipé d'un réfrigérant, d'une ampoule à introduction, d'un thermomètre et d'une arrivée d'azote, on introduit le 8-oxotricyclo[5.2.1.0^{2,6}]décane (22g, 0,15 mole) et 200ml de toluène et on porte au reflux. On ajoute le méthylate de sodium (9,72g, 0,18 mole). On laisse 20 minutes sous agitation. On ajoute ensuite goutte à goutte en 2 heures le para téréphtaldéhyde (8,04g, 0,06 mole) en solution dans un mélange de 50ml de toluène et 10ml de méthanol. On laisse au reflux pendant 3 heures. On refroidit et on verse dans l'eau. La phase organique est séchée et concentrée. Le produit brut obtenu est recristallisé dans 500ml d'un mélange 2 :3 dichlorométhane/éther isopropylique pour obtenir le 1,4-phénylène-bis-(méthylidène cétotricyclodécane) (4,2g, Rendement 81%) sous forme d'une poudre blanche. (Pf = 235-236°C).
- UV (solution dans CHCl₃) : λₘₐₓ = 340 nm, εₘₐₓ = 43 100, E1% = 1 080.
- Analyse élémentaire pour C₂₈H₃₀O₂
   - Calculé :C84,38 H7.59 08,03
   - Trouvé :C84,19 H7,68 08,14.

### Exemple 5

### Synthèse du 1,4-phénylène-bis-(3-méthylidène camphre)

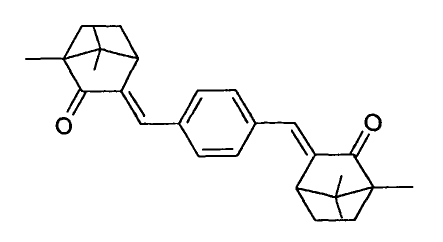

Dans un ballon tout équipé, on place le camphre (66,98g, 0,44mole) et le méthylate de sodium en poudre (23,76g, 0,44 mole) dans 100 ml de toluène sec. Le mélange est porté au reflux et laissé à cette température pendant 1 heure. On ajoute ensuite goutte à goutte en 2 heures le téréphtaldéhyde (26,8g, 0,2 mole) dissous à chaud dans un mélange de 100 ml de toluène sec, 10ml de pyridine et 16ml de méthanol. Le méthanol est distillé et on maintient à 110°C pendant 8 heures. On verse le mélange réactionnel dans la glace et on extrait à l'éther diéthylique. Le solide est recristallisé dans l'éther diéthylique pour obtenir, après séchage, le 1,4-phénylène-bis-(3-méthylidène camphre) (23g, Rendement 13%) sous forme d'une poudre blanche (Pf 234°C).
- UV (solution dans CHCl₃) : λₘₐₓ = 337 nm, εₘₐₓ = 38 720, E1% = 960.
- Analyse élémentaire pour C₂₈H₃₄O₂
   - Calculé : C83,54 H8,51 07,95
   - Trouvé : C83,54 H8,56 07,91

### Préparation d'une dispersion aqueuse du filtre :

On réalise une dispersion à 50 % dans l'eau du filtre insoluble en présence de 1% d'alkyl polyglucoside (APG) commercialisé par la société Henkel sous la marque Plantaren.

### Préparation de la pré-dispersion :

Dans un bécher de 50 ml on introduit de petites fractions de poudre dans le mélange eau + APG préalablement homogénéisé.
On laisse sous agitation aux ultra-sons pendant 45mn pour bien mouiller la poudre.

### Broyage:

Matériel utilisé : broyeur à billes de type Recht MM200 commercialisé par la société Recht, bol de 25 ml, 2 billes d'acier de 2 cm de diamètre.
On introduit la pré-dispersion obtenue précédemment dans le bol du broyeur contenant les billes. Le broyage est réalisé pendant 1 heure avec une fréquence d'agitation de 15.

### Caractérisation:

La dispersion obtenue à partir du composé préparé selon l'exemple 5 de l'invention est caractérisée à l'aide d'un granulomètre de type Mastersizer 2000 commercialisé par la société Malvern.
Le diamètre moyen de la distribution en nombre est : d = 0,77 µm pour le produit broyé une heure dans l'équipement utilisé.

### Exemple 6

### Synthèse du (2,3,5,6-tétraméthyl)-1,4-phénylène-bis-(3-méthylidène camphre)

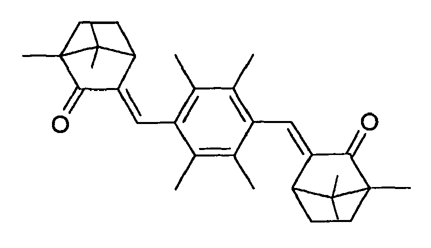

Dans un ballon tout équipé, on place le camphre (40g, 0,264mole), la soude en pastilles (9.6g, 0,24 mole) et le 2,3,5,6-tétraméthyl-téréphtaldéhyde (22,82g, 0,12 mole) dans 200ml de toluène sec. Le mélange est porté au reflux et laissé à cette température pendant 96 heures. On verse le mélange réactionnel dans la glace et on extrait au chloroforme. La phase organique est lavée à l'eau et séchée sur sulfate de sodium. Après filtration, séchage et passage sur silice (éluant : toluène), on obtient le (2,3,5,6-tétraméthyl)-1,4-phénylène-bis-(3-méthylidène camphre) (14,3g, Rendement 26%) sous forme d'une poudre blanche.
- UV (solution dans CHCl₃) : λₘₐₓ = 286 nm, εₘₐₓ = 12 200 , E1% = 270.
- Analyse élémentaire pour C₃₂H₄₂O₂
   - Calculé : C83,79 H9,23 07,05
   - Trouvé : C83,59 H9,24 06,98

### Exemple 7

### Synthèse de la 1,4-phénylène-bis-(2-méthylidène quinuclidinone)

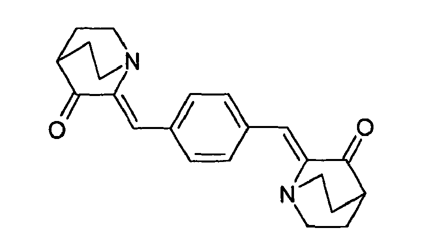

Dans un ballon tout équipé, on introduit le chlorhydrate de quinuclidinone (32g, 0,2 mole) dans 100ml d'eau. On y ajoute une solution de soude (64g dans 200ml d'eau ). On porte à 80°C sous azote et on ajoute en 30 minutes du téréphtaldéhyde (12,06g, 0,09 mole) en solution dans 150ml d'éthanol chaud. Un précipité jaune apparaît. On laisse au reflux pendant 4 heures. On refroidit, filtre et lave le solide à l'eau. On obtient, après séchage sous vide, la 1,4-phénylène-bis-(2-méthylidène quinuclidinone) (21,5g, Rendement 69%) sous forme d'une poudre jaune pâle. (Pf > 270°C).
- UV (solution dans DMSO) :
   λₘₐₓ = 346 nm, εₘₐₓ = 29 600, E1% = 850
   λₘₐₓ = 364 nm, εₘₐₓ = 25 000, E1% = 717.
- Analyse élémentaire pour C₂₀H₁₂N₂O₂
   - Calculé :C75,83 H6,94 N8,04 09,18
   - Trouvé :C75,45 H7,08 N8,00 09,37.

### Exemple 8

### Synthèse de la 2,5-bis-[4-(3-triméthylsilanyl-propyloxy)-benzylidène]-cyclopentanone

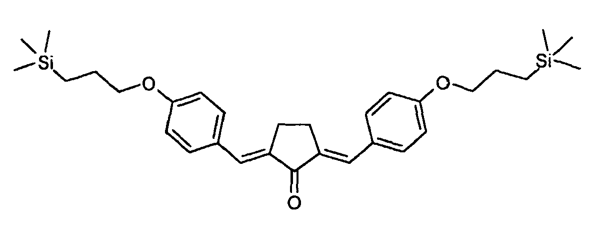

### a) Première étape: préparation du 4-(3-triméthylsilanyl-propyloxy)-benzaldéhyde :

Dans un mélange de 4-hydroxy benzaldéhyde (24,4 g, 0,2 mole) et de carbonate de potassium (30,4 g, 0,22 mole) dans 150 ml de DMF sec porté à 120°C sous azote, on ajoute goutte à goutte en 10 minutes du 3-chloropropyltriméthylsilane (33,14 g, 0,22 mole). On laisse 2 heures 30 minutes à 120-130°C. On refroidit et verse le mélange réactionnel dans de l'eau glacée. La phase aqueuse est extraite 3 fois au dichlorométhane. Les phases organiques sont séchées sur sulfate de sodium et concentrées sous vide. Après distillation sous vide (0,2 mmHg), on obtient 40,5g (Rendement : 86 %) de 4-(3-triméthylsilanyl-propyloxy)-benzaldéhyde sous forme d'une huile incolore distillant à 110-114°C et utilisée telle quelle dans l'étape suivante.

### b) deuxième étape: préparation du dérivé de l'exemple 8 :

Dans un mélange du produit précédent (26,2g, 0,105 mole) et de cyclopentanone (4,2 g, 0,05 mole) dans 200 ml de méthanol, sous azote, on ajoute à 35°C sous agitation goutte à goutte en 15 minutes 4,2 g de soude (0,105 mole) dissoute dans 22 ml d'eau. On porte au reflux pendant 6 heures. On refroidit, on filtre le précipité jaune obtenu et on le lave abondamment à l'eau. Après séchage, on obtient 25 g (Rendement : 96 %) du dérivé de l'exemple 8 sous forme d'un solide jaune.
- Point de fusion : 205-207°C.
- UV (solution dans CHCl₃) : λₘₐₓ = 391 nm, εₘₐₓ = 49 100, E1% = 940.
- Analyse élémentaire pour C₃₁H₄₄O₃Si₂
   - Calculé : C71,49 H8,51 Si10,78
   - Trouvé : C71,15 H8,78 Si10,47.

### Exemple de composition cosmétique

### Crème huile dans eau

| | |
|---|---|
| Mélange d'alcool cétylstéarylique et d'alcool cétylstéarylique oxyéthyléné (33 OE) 80/20 (SINNOWA® AO-HENKEL) | 7 g |
| Mélange de mono et distéarate de glycérol (CERASYNT® SD-V ISP) | 2 g |
| Alcool Cétylique | 1,5g |
| Polydiméthyl siloxane (DOW CORNING 200 FLUID® -DOW COIZNING) | 1,5 g |
| Benzoate d'alcools en C12/C15 (WITCONOL TN - WITCO) | 15 g |
| 1,4-Phénylène-bis-(3-méthylidène) camphre broyé en dispersion aqueuse à 50% , selon l'exemple 5 | 10 g |
| Glycérine | 20 g |
| Conservateurs | qs |
| Eau déminéralisée qsp | 100 g |

On chauffe séparément la phase grasse et la phase aqueuse à 70 °C, puis on introduit la phase huileuse dans la phase aqueuse sous agitation. On maintient l'agitation pendant environ 15 minutes et on laisse refroidir sous agitation lente jusqu'à retour de la composition à température ambiante.
L'efficacité de cette composition est évaluée in vitro à l'aide d'un spectroradiomètre de type SPF 290 commercialisé par la société Optometrics et selon la méthode de Diffey et Robson (B.L. Diffey et al., J. Soc. Cosmet. Chem., 40, 127 - 133 (1989)) sur plaque de quartz revêtue d'un ruban Transpore® . Le facteur de protection solaire (FPS) est égal à 3,0 ± 0,3.

## Revendications

1. Composé organique insoluble de type aryl vinylène cétone filtrant le rayonnement UV et se présentant sous forme de particules ayant une taille moyenne comprise entre 10 nm et 5 µm, et correspondant à l'une des formules (I) et (II) suivantes : 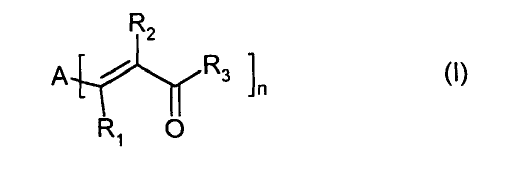 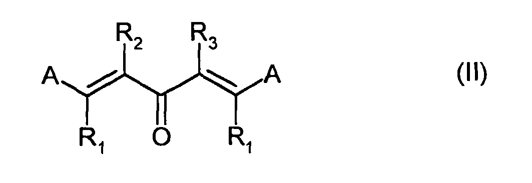 dans lesquelles :
n = 1 ou 2,
A, dans la formule (I) lorsque n=1 ou dans la formule (II), est un radical aryle choisi parmi les formules (a) à (d) suivantes, ou dans la formule (I) lorsque n=2, est un radical choisi parmi les formules (e) à (h) suivantes : 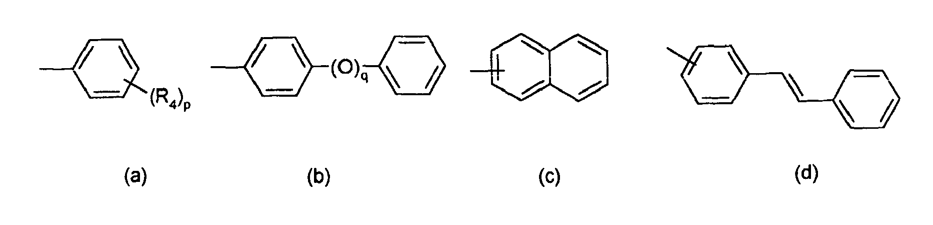 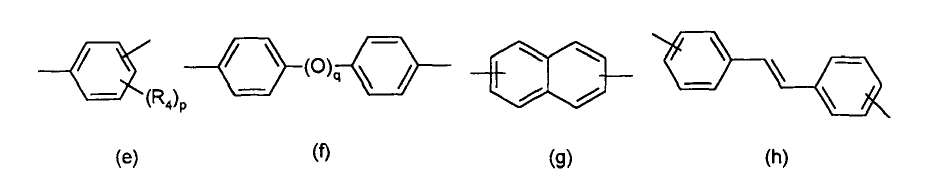
dans lesquelles :
chacun des symboles R₄ représente indépendamment un groupe OH, un atome d'halogène, un groupe alkyle en C₁₋₆, linéaire ou ramifié et contenant éventuellement un atome de silicium, un groupe alcoxy en C₁₋₆, linéaire ou ramifié et contenant éventuellement un atome de silicium, un groupe alcoxycarbonyle en C₁₋₅, linéaire ou ramifié, ou un groupe alkylsulfonamide en C₁₋₆, linéaire ou ramifié et contenant éventuellement un atome de silicium ou une fonction aminoacide,
p représente un nombre entier compris entre 0 et 4 inclus,
q représente 0 ou 1,
R₁ représente l'hydrogène ou un groupe OH,
R₂ représente l'hydrogène, un groupe alkyle en C₁₋₆, linéaire ou ramifié et contenant éventuellement un atome de silicium, un groupe cyano, un groupe alkylsulfonyle en C₁₋₆, un groupe phénylsulfonyle,
R₃ représente un groupe alkyle en C₁₋₆, linéaire ou ramifié et contenant éventuellement un atome de silicium ou un groupe phényle pouvant former un bicycle et éventuellement substitué par un ou deux radicaux R₄,
ou R₂ et R₃ forment ensemble un reste hydrocarboné en C₂₋₁₀ monocyclique, bicyclique ou tricylique, éventuellement interrompu par un ou des atomes d'azote, de soufre et d'oxygène et pouvant contenir un autre carbonyle, et éventuellement substitué par un groupe alkylsulfonamide en C₁₋C₈, linéaire ou ramifié, et contenant éventuellement un atome de silicium ou une fonction aminoacide ; à condition que lorsque n=1, R₂ et R₃ ne forment pas un noyau camphre.

2. Composé organique insoluble de formule (I) dans laquelle n=1 selon la revendication 1, **caractérisé par le fait qu'**il est choisi parmi les styryl cétones, benzylidène cinéoles, benzylidène chromanones, benzylidène thiochromanones, benzylidène quinuclidinones, benzylidène cycloalcanones, benzylidène hydantoïnes, benzylidène indanones, benzylidène tétralones, benzylidène furanones, benzylidène benzofuranones, benzylidène indanediones, benzylidène benzothiofuranones, benzylidène barbituriques, benzylidène pyrazolones, benzylidène imidazolones, les chalcones et les benzylidène ones.

3. Composé organique insoluble de formule (I) dans laquelle n=2 selon la revendication 1, **caractérisé par le fait qu'**il est choisi parmi les phénylène bis méthylidène-nor-camphres, phénylène bis méthylidène camphres, phénylène bis méthylidène camphre sulfonamides, phénylène bis méthylidène cinéoles, phénylène bis méthylidène cétotricyclodécanes, les bis phénylène alkylène cétones, les phénylène bis méthylidène furanones et les phénylène bis méthylidène quinuclidinones.

4. Composé organique insoluble de formule (II) selon la revendication 1, **caractérisé par le fait qu'**il est choisi parmi les bis benzylidène cycloalcanones et les gamma pyrones.

5. Composé organique insoluble selon l'une quelconque des revendications précédentes, **caractérisé par le fait qu'**il se présente sous forme de particules ayant une taille moyenne comprise entre 10 nm et 2 µm, de préférence entre 20 nm et 2 µm.

6. Composition cosmétique contenant au moins un composé organique insoluble filtrant le rayonnement UV selon l'une quelconque des revendications précédentes, dans un support cosmétiquement acceptable.

7. Composition cosmétique selon la revendication 6, **caractérisée par le fait que** la fraction du composé organique insoluble filtrant le rayonnement UV est comprise entre 0,1 % et 15 % en poids et de préférence entre 0,2 et 10 % en poids, par rapport au poids total de la composition cosmétique.

8. Composition cosmétique selon l'une des revendications 6 et 7, **caractérisée par le fait qu'**elle contient en outre un ou plusieurs filtres solaires organiques actifs dans le domaine des UV-A et/ou UV-B, différents de ceux selon les revendications 1 à 5.

9. Composition cosmétique selon la revendication 8, **caractérisée par le fait que** les filtres solaires organiques sont choisis parmi les suivants : Ethylhexyl Salicylate, Butyl Methoxydibenzoylmethane, Ethylhexyl Methoxycinnamate, Octocrylene, Phenylbenzimidazole Sulfonic Acid, Terephthalylidene Dicamphor Sulfonic Acid, Benzophenone-3, Benzophenone-4, Benzophenone-5, 4-Methylbenzylidene Camphor, Benzimidazilate, Aniso-triazine, Ethylhexyl Triazone, Diethylhexyl Butamido Triazone, 2,4,6-tris(4'-aminobenzalmalonate de di-isobutyle) s-triazine, Methylene bis-Benzotriazolyl Tetramethylbutylphenol, Drometrizole Trisiloxane, et des mélanges de ceux-ci.

10. Composition cosmétique selon l'une quelconque des revendications 6 à 9, **caractérisée par le fait qu'**elle contient en outre un ou plusieurs pigments minéraux.

11. Composition cosmétique selon la revendication 10, **caractérisée par le fait que** les pigments minéraux sont choisis parmi les nanopigments d'oxydes métalliques tels que l'oxyde de titane, l'oxyde de fer, l'oxyde de zinc, l'oxyde de zirconium et l'oxyde de cérium, éventuellement enrobés, et leurs mélanges.

12. Composition cosmétique selon la revendication 11, **caractérisée par le fait que** les nanopigments ont une taille moyenne de particules comprise entre 5 nm et 100 nm, de préférence entre 10 nm et 50 nm.

13. Composition cosmétique selon l'une quelconque des revendications 6 à 12, **caractérisée par le fait qu'**elle contient en outre au moins un agent de bronzage et/ou de brunissage artificiel de la peau.

14. Composition cosmétique selon l'une quelconque des revendications 6 à 13, **caractérisée par le fait qu'**elle contient en outre un ou plusieurs adjuvants de formulation choisis parmi les corps gras, les solvants organiques physiologiquement acceptables, les agents épaississants, les agents antioxydants, les agents opacifiants, les agents stabilisants, les agents anti-mousse, les parfums, les agents conservateurs, les charges, les agents séquestrants, les agents propulseurs, les agents d'ajustement du pH, les colorants et leurs mélanges.

15. Composition cosmétique selon l'une quelconque des revendications 6 à 14, **caractérisée par le fait qu'**elle contient en outre un ou plusieurs principes actifs cosmétiques choisis parmi les vitamines, les agents adoucissants, les hydroxyacides, les agents hydratants, les agents émollients, les agents anti-radicalaires, les antagonistes de substance P, les agents anti-inflammatoires et des mélanges de ces composés.

16. Composition cosmétique selon l'une quelconque des revendications 6 à 15, **caractérisée par le fait qu'**elle constitue une composition cosmétique destinée à protéger la peau ou les cheveux, une composition anti-solaire ou un produit de maquillage.

17. Utilisation d'un ou de plusieurs composés selon l'une quelconque des revendications 1 à 5 pour la protection de matériaux sensibles au rayonnement UV, en particulier au rayonnement solaire

18. Composition destinée à protéger un matériau sensible au rayonnement UV, en particulier au rayonnement solaire, **caractérisée par le fait qu'**elle comprend une quantité efficace d'au moins un composé selon l'une quelconque des revendications 1 à 5.

19. Utilisation d'un ou de plusieurs composés selon l'une quelconque des revendications 1 à 5, pour la préparation d'un médicament destiné à prévenir les effets néfastes des rayonnements UV.

20. Procédé non-thérapeutique de protection de la peau ou des cheveux contre le rayonnement ultraviolet, en particulier le rayonnement solaire, **caractérisé par le fait qu'**il consiste à appliquer sur la peau ou les cheveux une quantité efficace d'une composition cosmétique selon l'une quelconque des revendications 6 à 16 ou d'un composé selon l'une quelconque des revendications 1 à 5.

21. Procédé non thérapeutique de contrôle de la variation de la couleur de la peau due au rayonnement UV, **caractérisé par le fait qu'**il consiste à appliquer sur la peau une quantité efficace d'une composition cosmétique selon l'une quelconque des revendications 6 à 15, ou d'un composé selon l'une quelconque des revendications 1 à 5.

## Claims

1. Insoluble organic compound of aryl vinylene ketone type for screening out Uv radiation, which is in the form of particles with an average size of between 10 nm and 5 µm, and corresponding to one of the formulae (I) and (II) below: 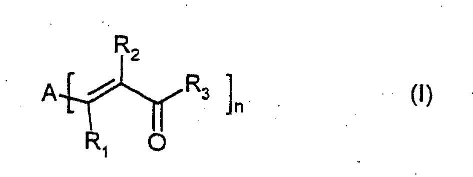 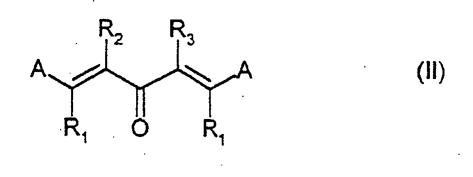 in which:
n=1 or 2,
A, in formula (I) when n=1 or in formula (II), is an aryl radical chosen from formulae (a) to (d) below, or in formula (I) when n=2, is a radical chosen from formulae (e) to (h) below: 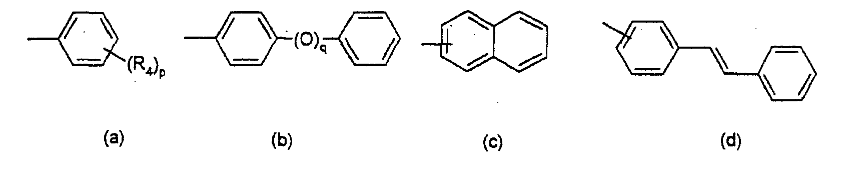 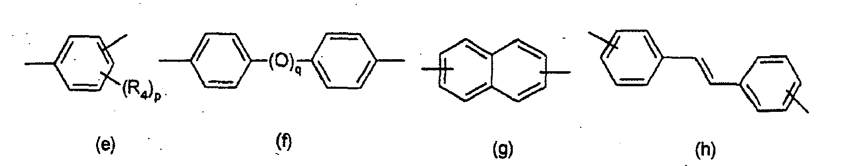
in which:
each of the symbols R₄ independently represents an OH group, a halogen atom, a linear or branched C₁₋₆ alkyl group optionally containing a silicon atom, a linear or branched C₁₋₆ alkoxy group optionally containing a silicon atom, a linear or branched C₁₋₅ alkoxycarbonyl group, or a linear or branched C₁₋₆ alkylsulphonamide group optionally containing a silicon atom or an amino acid function,
p represents an integer between 0 and 4 inclusive,
q represents 0 or 1,
R₁ represents hydrogen or an OH group,
R₂ represents hydrogen, a linear or branched C₁₋₆ alkyl group optionally containing a silicon atom, a cyano group, a C₁₋₆ alkylsulphonyl group or a phenylsulphonyl group,
R₃ represents a linear or branched C₁₋₆ alkyl group optionally containing a silicon atom or a phenyl group that can form a bicycle and optionally substituted with one or two radicals R₄,
or R₂ and R₃ together form a monocyclic, bicyclic or tricyclic C₂₋₁₀ hydrocarbon residue optionally interrupted with one or more nitrogen, sulphur and oxygen atoms and possibly containing another carbonyl, and optionally substituted with a linear or branched C₁-C₈ alkylsulphonamide group, and optionally containing a silicon atom or an amino acid function; on condition that, when n=1, R₂ and R₃ do not form a camphor nucleus.

2. Insoluble organic compound of formula (I) in which n=1 according to Claim 1, **characterized in that** it is chosen from styryl ketones, benzylidene cineoles, benzylidene chromanones, benzylidene thiochromanones, benzylidene quinuclidinones,benzylidene cycloalkanones, benzylidene hydantoins, benzylidene indanones, benzylidene tetralones, benzylidene furanones, benzylidene benzofuranones, benzylidene indanediones, benzylidene benzothiofuranones, benzylidene barbiturics, benzylidene pyrazolones, benzylidene imidazolones, chalcones and benzylidene ones.

3. Insoluble organic compound of formula (I) in which n=2 according to Claim 1, **characterized in that** it is chosen from phenylenebis(methylidene-nor-camphors), phenylenebis(methylidenecamphors), phenylenebis-(methylidenecamphorsulphonamides), phenylenebis-(methylidene cineoles), phenylenebis(methylidene ketotricyclodecanes), bis(phenylene alkylene ketones), phenylenebis(methylidene furanones) and phenylenebis-(methylidene quinuclidiriones).

4. Insoluble organic compound of formula (II) according to Claim 1, **characterized in that** it is chosen from bis(benzylidene cycloalkanones) and gammapyrones.

5. Insoluble organic compound according to any one of the preceding claims, **characterized in that** it is in the form of particles with an average size of between 10 nm and 2 µm and preferably between 20 nm and 2 µm.

6. Cosmetic composition containing at least one insoluble organic compound for screening out UV radiation according to any one of the preceding claims, in a cosmetically acceptable support.

7. Cosmetic composition according to Claim 6, **characterized in that** the fraction of the insoluble organic compound for screening out UV radiation is between 0.1% and 15% by weight and preferably between 0.2% and 10% by weight, relative to the total weight of the cosmetic composition.

8. Cosmetic composition according to either of Claims 6 and 7, **characterized in that** it also contains one or more organic sunscreens that are active in the UV-A and/or UV-B range, other than those according to Claims 1 to 5.

9. Cosmetic composition according to Claim 8, **characterized in that** the organic sunscreens are chosen from the following: ethylhexyl salicylate, butyl methoxydibenzoylmethane, ethylhexyl methoxycinnamate, octocrylene, phenylbenzimidazolesulphonic acid, terephthalylidenedicamphorsulphonic acid, benzophenone-3, benzophenone-4, benzophenone-5, 4-methylbenzylidenecamphor, benzimidazilate, anisotriazine, ethylhexyltriazone, diethylhexylbutamidotriazone, 2 ,4 , 6-tris (diisobutyl 4'-aminobenzalmalonate) s-triazine, methylenebis(benzotriazolyl)tetramethylbutylphenol, drometrizole trisiloxane, and mixtures thereof.

10. Cosmetic composition according to any one of Claims 6 to 9, **characterized in that** it also contains one or more mineral pigments.

11. Cosmetic composition according to Claim 10, **characterized in that** the mineral pigments are chosen from nanopigments of metal oxides such as titanium oxide, iron oxide, zinc oxide, zirconium oxide and cerium oxide, that are optionally ,coated, and mixtures thereof.

12. Cosmetic composition according to Claim 11, **characterized in that** the nanopigments have a mean particle size of between 5 nm and 100 nm and preferably between 10 nm and 50 nm.

13. Cosmetic composition according to any one of Claims 6 to 12, **characterized in that** it also contains at least one agent for artificially tanning and/or browning the skin.

14. Cosmetic composition according to any one of Claims 6 to 13, **characterized in that** it also contains one or more formulation adjuvants chosen from fatty substances, physiologically acceptable organic solvents, thickeners, antioxidants, opacifiers, stabilizers, antifoams, fragrances, preserving agents, fillers, sequestering agents, propellants, pH modifiers and colorants, and mixtures thereof.

15. Cosmetic composition according to any one of Claims 6 to 14, **characterized in that** it also contains one or more cosmetic active principles chosen from vitamins, softeners, hydroxy acids, moisturizers, emollients, free-radical scavengers, substance P antagonists, anti-inflammatories and mixtures of these compounds.

16. Cosmetic composition according to any one of Claims 6 to 15, **characterized in that** it constitutes a cosmetic composition for protecting the skin or the hair, an antisun composition or a makeup product.

17. Use of one or more compounds according to any one of Claims 1 to 5 for protecting materials that are sensitive to UV radiation, in particular solar radiation.

18. Composition for protecting a material that is sensitive to UV radiation, in particular solar radiation, **characterized in that** it comprises an effective amount of at least one compound according to any one of Claims 1 to 5.

19. Use of one or more compounds according to any one of Claims 1 to 5, for the preparation of a medicinal product for preventing the harmful effects of UV radiation.

20. Non-therapeutic process for protecting the skin or the hair against ultraviolet radiation, in particular solar radiation, **characterized in that** it consists in applying to the skin or the hair an effective amount of a cosmetic composition according to any one of Claims 6 to 16, or of a compound according to any one of Claims 1 to 5.

21. Non-therapeutic process for controlling the variation in the colour of the skin caused by UV radiation, **characterized in that** it consists in applying to the skin an effective amount of a cosmetic composition according to any one of Claims 6 to 15, or of a compound according to any one of Claims 1 to 5.

## Patentansprüche

1. Unlösliche organische Verbindung vom Typ Arylvinylenketon, die die UV-Strahlung filtert und in Form von Partikeln mit einer mittleren Größe von 10 nm bis 5 µm vorliegt und einer der folgenden Formeln (I) und (II) entspricht: 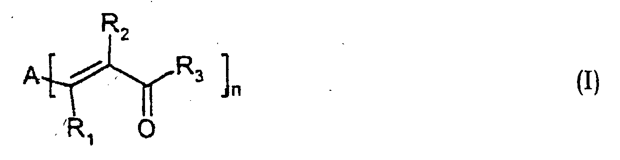 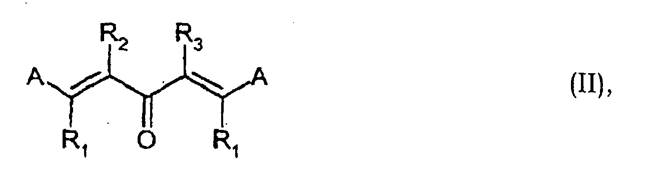 worin bedeuten:
· n = 1 oder 2,
· A in der Formel (I), wenn n 1 bedeutet, oder in der Formel (II) eine Arylgruppe, die unter den folgenden Formeln (a) bis (d) ausgewählt ist, oder in der Formel (I), wenn n 2 bedeutet, eine Gruppe, die unter den folgenden Formeln (e) bis (h) ausgewählt ist: 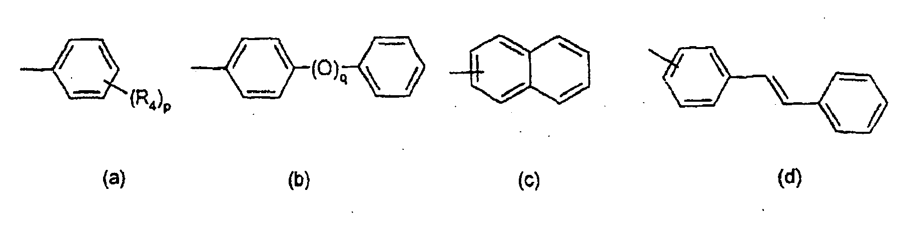 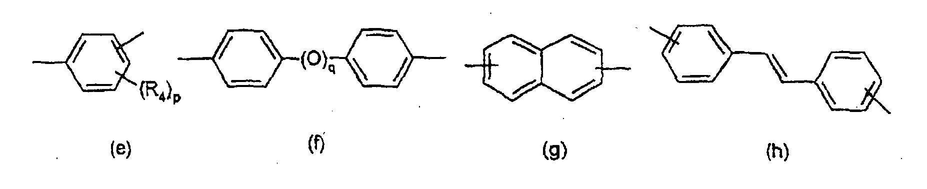
worin bedeuten:
- die Symbole R₄ jeweils unabhängig eine Gruppe OH, ein Halogenatom, eine geradkettige oder verzweigte C₁₋₆₋Alkylgruppe, die gegebenenfalls ein Siliciumatom enthält, eine geradkettige oder verzweigte C₁₋₆-Alkoxygruppe, die gegebenenfalls ein Siliciumatom enthält, eine geradkettige oder verzweigte C₁₋₅-Alkoxycarbonylgruppe oder eine geradkettige oder verzweigte C₁₋₆-Alkylsulfonamidgruppe, die gegebenenfalls ein Siliciumatom enthält, oder eine Aminosäurefunktion,
- p 0 oder eine ganze Zahl von 1 bis 4,
- q 0 oder 1,
· R₁ Wasserstoff oder eine OH-Gruppe,
· R₂ Wasserstoff, eine geradkettige oder verzweigte C₁₋₆-Alkylgruppe, die gegebenenfalls eine Siliciumatom enthält, eine Cyanogruppe, eine C₁₋₆-Alkylsulfonylgruppe oder eine Phenylsulfonylgrüppe,
· R₃ eine geradkettige oder verzweigte C₁₋₆-Alkylgruppe, die gegebenenfalls ein Siliciumatom enthält, oder eine Phenylgruppe, die einen Bicyclus bilden und gegebenenfalls mit einer oder zwei Gruppen R₄ substituiert sein kann,
· oder R₂ und R₃ bilden gemeinsam einen monocyclischen, bicyclischen oder tricyclischen C₂₋₁₀-Kohlenwasserstoffrest, der gegebenenfalls mit einem oder mehreren Stickstoffatomen, Schwefelatomen und Sauerstoffatomen unterbrochen ist und gegebenenfalls ein weiteres Carbonyl enthalten kann und gegebenenfalls mit einer geradkettigen oder verzweigten C₁₋₈₋Alkylsulfonamidgruppe substituiert ist und gegebenenfalls ein Siliciumatom oder eine Aminosäurefunktion enthalten kann; mit der Maßgabe, dass R₂ und R₃ keinen Campherring bilden, wenn n 1 ist.

2. Unlösliche organische Verbindung der Formel (I) mit n = 1 gemäß Anspruch 1, **dadurch gekennzeichnet, dass** sie unter den Styrylketonen, Benzylidencineolen, Benzylidenchromanonen, Benzylidenthiochromanonen, Benzylidenchinuclidinonen, Benzylidencycloalkanonen, Benzylidenhydantoinen, Benzylidenindanonen, Benzylidentetralonen, Benzylidenfuranonen, Benzylidenbenzofuranonen, Benzylidenindandionen, Benzylidenbenzothiofuranonen, Benzylidenbarbiturverbindungen, Benzylidenpyrazolonen, Benzylidenimidazolonen, Chalkonen und Benzylidenonen ausgewählt ist.

3. Unlösliche organische Verbindung der Formel (I) mit n = 2 gemäß Anspruch 1, **dadurch gekennzeichnet, dass** sie unter den Phenylen-bis-methyliden-norcamphern, Phenylen-bis-methylidencamphern, Phenylen-bis-methylidencamphersulfonamiden, Phenylen-bis-methylidencineolen, Phenylen-bis-methyliden-ketotricyclodekanen, Bisphenylenalkylenketonen, Phenylen-bismethylidenfuranonen und Phenylen-bis-methylidenchinuclidinonen ausgewählt ist.

4. Unlösliche organische Verbindung der Formel (II) nach Anspruch 1, **dadurch gekennzeichnet, dass** sie unter den Bisbenzylidencycloalkanonen und γ-Pyronen ausgewählt ist.

5. Unlösliche organische Verbindung nach einem der vorhergehenden Ansprüche, **dadurch gekennzeichnet, dass** sie in Form von Partikeln mit einer mittleren Größe von 10 nm bis 2 µm und vorzugsweise 20 nm bis 2 µm vorliegt.

6. Kosmetische Zusammensetzung, die mindestens eine unlösliche organische Verbindung, die die UV-Strahlung filtert, nach einem der vorhergehenden Ansprüche in einem kosmetisch akzeptablen Träger enthält.

7. Kosmetische Zusammensetzung nach Anspruch 6, **dadurch gekennzeichnet, dass** der Anteil der unlöslichen organischen Verbindung, die die UV-Strahlung filtert, im Bereich von 0,1 bis 15 Gew.-% und vorzugsweise 0,2 bis 10 Gew.-%, bezogen auf das Gesamtgewicht der kosmetischen Zusammensetzung, liegt.

8. Kosmetische Zusammensetzung nach einem der Ansprüche 6 und 7, **dadurch gekennzeichnet, dass** sie ferner ein oder mehrere organische Sonnenschutzfilter enthält, die im UV-A- und/oder UV/B-Bereich wirksam sind und von den Filtern nach den Ansprüchen 1 bis 5 verschieden sind.

9. Kosmetische Zusammensetzung nach Anspruch 8, **dadurch gekennzeichnet, dass** die organischen Sonnenschutzfilter unter den folgenden Verbindungen ausgewählt wind: Ethylhexyl Salicylate, Butyl Methoxydibenzoylmethane, Ethylhexyl Methoxycinnamate, Octocrylene, Phenylbenzimidazole Sulfonic Acid, Terephthalylidene Dicamphor Sulfonic Acid, Benzophenone-3, Benzophenone-4, Benzophenone-5, 4-Methylbenzylidene Camphor, Benzimidazilate, Aniso-triazine, Ethylhexyl Triazone, Diethylhexyl Butamido Triazone, 2,4,6-Tris-(diisobutyl-4'-aminobenzalmalonat)-s-triazin, Methylene bis-Benzotriazolyl Tetramethylbutylphenol, Drometrizole Trisiloxane und den Gemischen dieser Verbindungen.

10. Kosmetische Zusammensetzung nach einem der Ansprüche 6 bis 9, **dadurch gekennzeichnet, dass** sie ferner ein oder mehrere anorganische Pigmente enthält.

11. Kosmetische Zusammensetzung nach Anspruch 10, **dadurch gekennzeichnet, dass** die anorganischen Pigmente unter den Nanopigmenten von Metalloxiden, wie Titanoxid, Eisenoxid, Zinkoxid, Zirconiumoxid und Ceroxid, die gegebenenfalls umhüllt sind, und deren Gemischen ausgewählt sind.

12. Kosmetische Zusammensetzung nach Anspruch 11, **dadurch gekennzeichnet, dass** die Nanopigmente eine mittlere Partikelgröße von 5 bis 100 nm und vorzugsweise 10 bis 50 nm aufweisen.

13. Kosmetische Zusammensetzung nach einem der Ansprüche 6 bis 12, **dadurch gekennzeichnet, dass** sie ferner mindestens ein Bräunungsmittel und/oder Mittel zur künstlichen Braunfärbung der Haut enthält.

14. Kosmetische Zusammensetzung nach einem der Ansprüche 6 bis 13, **dadurch gekennzeichnet, dass** sie ferner einen oder mehrere Hilfsstoffe für die Formulierung enthält, die unter den Fettsubstanzen, physiologisch akzeptablen organischen Lösungsmitteln, Verdickungsmitteln, Antioxidantien, Trübungsmitteln, Stabilisatoren, Schaumverhütungsmitteln, Parfums, Konservierungsmitteln, Füllstoffen, Maskierungsmitteln, Triebmitteln, pH-Reglern, Farbmitteln und deren Gemischen ausgewählt sind.

15. Kosmetische Zusammensetzung nach einem der Ansprüche 6 bis 14, **dadurch gekennzeichnet, dass** sie ferner einen oder mehrere kosmetische Wirkstoffe enthält, die unter den Vitaminen, beruhigenden Stoffen, Hydroxysäuren, Hydratisierungsmitteln, Emollientien, Radikalfängern für freie Radikale, Substanz P-Antagonisten, entzündungshemmenden Wirkstoffen und den Gemischen dieser Verbindungen ausgewählt sind.

16. Kosmetische Zusammensetzung nach einem der Ansprüche 6 bis 15, **dadurch gekennzeichnet, dass** sie eine kosmetische Zusammensetzung, die für den Schutz der Haut oder der Haare vorgesehen ist, ein Sonnenschutzmittel oder ein Produkt zum Schminken ist.

17. Verwendung einer oder mehrerer Verbindungen nach einem der Ansprüche 1 bis 5 zum Schutz von UV-empfindlichen Materialien und insbesondere Materialien, die gegenüber Sonnenlicht empfindlich sind.

18. Zusammensetzung, die zum Schutz eines UV-empfindlichen Materials und insbesondere eines Materials, das gegenüber Sonnenlicht empfindlich ist, vorgesehen ist, **dadurch gekennzeichnet, dass** sie mindestens eine Verbindung nach einem der Ansprüche 1 bis 5 in einer wirksamen Menge enthält.

19. Verwendung einer oder mehrerer Verbindungen nach einem der Ansprüche 1 bis 5 für die Herstellung eines Arzneimittels, das für die Vorbeugung gegen die schädlichen Wirkungen der UV-Strahlung vorgesehen ist.

20. Nichttherapeutisches Verfahren zum Schutz der Haut oder der Haare gegen UV-Strahlung und insbesondere Sonnenlicht, **dadurch gekennzeichnet, dass** es darin besteht, auf die Haut oder die Haare eine wirksame Menge einer kosmetischen Zusammensetzung nach einem der Ansprüche 6 bis 16 oder einer Verbindung nach einem der Ansprüche 1 bis 5 aufzubringen.

21. Nichttherapeutisches Verfahren zur Kontrolle der durch UV-Strahlung verursachten Farbänderung der Haut, **dadurch gekennzeichnet, dass** es darin besteht, auf die Haut eine wirksame Menge einer kosmetischen Zusammensetzung nach einem der Ansprüche 6 bis 15 oder einer Verbindung nach einem der Ansprüche 1 bis 5 aufzutragen.
